# EUROPEAN PATENT SPECIFICATION

(11) **EP 4 062 867 B1**
(45) Date of publication and mention of the grant of the patent: **07.08.2024**
(21) Application number: 22163219.3
(22) Date of filing: 21.03.2022
(51) Int. Cl.: A61C 19/00, A61L 2/00, B65D 83/14, F16B 2/02, F16L 37/00

(54) **DEVICE FOR LUBRICATINGING AND OPTIONALLY CLEANING AND DISINFECTING DENTAL HANDPIECES**
VORRICHTUNG ZUM SCHMIEREN UND GEGEBENENFALLS REINIGEN UND DESINFIZIEREN VON ZAHNÄRZTLICHEN HANDSTÜCKEN
DISPOSITIF DE LUBRIFICATION ET DE NETTOYAGE ET DE DÉSINFECTION ÉVENTUELS DE PIÈCES À MAIN DENTAIRES

(30) Priority: 23.03.2021 IT 202100006911
(43) Date of publication of application: 28.09.2022
(73) Proprietor: CEFLA Società Cooperativa, 40026 Imola (BO) (IT)
(72) Inventor: BORSARI, Valerio, 40026 Imola (IT); MATTIUZZO, Giulio, 40026 Imola (IT)
(74) Representative: Karaghiosoff, Giorgio Alessandro

(56) References cited:
- EP-A1- 1 652 488
- WO-A1-2006/039509

## Description

The present invention relates to two desktop devices, in a first simple embodiment for lubricating, and in a second more sophisticated embodiment for cleaning, disinfecting and lubricating dental handpieces. In particular, such devices are provided with some innovative solutions which markedly improve their usability by the human operators appointed to cleaning and disinfecting the dental handpieces that were used on a patient.

In the art, there are known different kinds of rotary or vibratory handpieces, which can be mounted on known dental treatment units or known stand-alone desktop units:
- turbines (rotatory handpieces);
- contrangles (rotatory handpieces);
- dental scalers, mostly piezoelectric (vibratory handpieces).

It is worth stating that vibratory handpieces do not need lubrication.

Said rotary and vibratory handpieces share come common features:
- On such handpieces accessories are mounted (tips, burs, prophy cups, etc.);
- Said handpieces are supplied with water and electric energy; in the case of turbines with water, electric energy and air;
- Said handpieces are provided with internal channels for supplying water and optionally air, whose internal cleaning cannot be ensured by autoclaving only.

For connecting said handpieces to dental treatment units or to stand-alone desktop devices, often a connection of the kind plug-and-socket six holes Midwest connection is employed, which is a standard in this field.

Said handpieces, which can be re-used on a plurality of patients, must be treated after their use on a single patient, in order to prevent the transmission of infective pathologies from a patient to the subsequent patient (cross infection). In particular, said accessories are disposable or are autoclaved, while the handpieces are autoclaved separately from their accessories.

All the following operations are performed by a human operator. A typical work flow is:
a) Disconnection of the handpiece from the dental treatment unit or stand-alone desktop device;
b) Removal of the accessory from its respective handpiece;
c) External manual cleaning of said handpiece;
d) When needed, internal cleaning and disinfection of the handpiece;
e) Lubricating the handpiece;
f) Bagging the handpiece;
g) Autoclave sterilization of the handpiece.

In the art, substantially two kinds of devices for the maintenance of dental handpieces have been known for a long time; they can perform the steps d) and e) of the workflow:
- A first, simpler kind of device, which can perform just the lubrication of said handpieces.
   E.g. a document of the known art is FR2340714 of Kaltenbach & Voigt, published in 1977. Such document describes a desktop device for the maintenance of medical instruments, in particular dental instruments, provided with mobile assembly accessories, through a maintenance handpiece to be applied to the instrument, which is connected to a big-volume container containing a maintenance medium under pressure, e.g. oil, and is provided with a supplying nozzle which can be opened and closed through a valve arranged in the maintenance handpiece.
- A second, more sophisticated kind of device, capable of performing the lubrication and also the cleaning and the disinfection of said handpieces. E.g. EP0056791A2 of Medical Engineering Consulting Grifo, published in 1982, describes a cleaning device for dental instruments, in particular, turbines and the like, by means of liquid jets, disinfectant media and air, comprising a box-like container, disinfecting means and air, including a chamber provided with an entrance opening which opens in the top wall of said container and through which the instrument to be washed is introduced in the inside of said chamber, by means of jets of compressed air and water dispensed by oriented nozzles, while other nozzles placed in the chamber downstream of the nozzles or in a separated chamber having an entrance orifice, deliver a mixture of air and disinfectant medium for drying up the instrument.

Said devices for Lubricating (for the sake of brevity L devices in the following), or for the Cleaning, Disinfection Lubrication (for the sake of brevity CDL devices in the following) are typically desktop devices, provided with specific reservoirs for the cleaning, disinfecting and lubricating means.

A further document of the known art is DE19913945A1 of Kaltenbach & Voigt, describing a device wherein the handpieces are connected to the device inside a closed chamber by a lid, and wherein the connections provided for the handpieces can be rotated. In particular, there is provided a first functional position wherein said handpieces are oriented downwards, and a second functional position wherein said handpieces are rotated with respect to the first functional position of an angle W1 of 45°-90°, so that handpieces can be connected or removed more easily by a human operator. Said rotation is performed by an actuator connected to said lid, so that, when said lid is opened, said connections are automatically in said angulated position W1.

A further document of the known art is US4486174A1 of Kaltenbach & Voigt, which describes a device for supplying a cleaning and/or lubricating means, contained in a container under pressure (spray can), which is connected to dental handpieces.

Document EP1652488A1 of Morita discloses a tabletop device for cleaning, disinfecting and lubricating dental handpieces which comprises connectors for coupling said handpieces, which connectors are placed inside a chamber which can be opened and in which said connectors are provided with a Midwest connector; the said devices further comprising an adapter to be interposed between the said contra-angles and the said connectors; said adapter being provided with a cylindrical shape; the first end of the said adapter being provided with a connector for the said contra-angle while the second end of the said adapter being provided with a Midwest connector.

Also document WO2006039509 A1 of Dentsply discloses a tabletop device for cleaning, sterilizing and lubricating dental handpieces which comprises an adapter for contra-angle to be interposed between the contra-angles and the connectors.

The said adapters which are shown as an example in figure 5 as originally filed are well known in the art. Nevertheless, the adapters described in EP1652488A1 and WO2006039509A1 show only static passageways for the cleaning and/or the sterilizing and/or the lubricating fluids but do not show any mechanism driven by the pressure of the said fluids which is able to generate dynamical behaviours of the adapter and of the fluids in order to optimize the distribution of the said fluids in particular on the gears of the contra-angles or of other rotational handpieces.

The present invention aims to provide two apparatuses, in a first embodiment an L device, and in a second embodiment a CDL device, provided of an improved usability by human operators.

This object is achieved by an apparatus having the features of the independent claims. Advantageous embodiment and refinements are specified in the claims dependent thereon.

The devices according to the present invention are provided in two embodiments:
I. A first simpler and cheaper embodiment L, capable of lubricating only rotary handpieces (contrangles and turbines);
II. A second more sophisticated and expensive embodiment CDL, capable of cleaning, disinfecting and lubricating rotary and vibratory handpieces.

The L and CDL devices are provided with three innovative features, which are independent:
a) A mechanical block for connecting handpieces (rotary and/or rotary or vibratory), which can rotate around an axis parallel to ground between two extreme positions, the first being at 0° and the second forming an angle ranging 45-90° with respect to the first position. All the connections rotate at the same time between said 0° position and said 45-90° position passing through all the intermediate positions.
b) The housing of the spray can of the lubricating means is made for housing spray cans having a standard 1-inch collar.
c) The CDL device only is provided with an accessory for contrangles which is interposed between the Midwest connection of the contrangle and the connector for handpieces; said accessory allows a rotation of the contrangle internal gears.

It is worth mentioning that cleaning and lubricating are performed in the inside of the handpieces only, while both the inside and the outside of the handpieces undergo a disinfection. Between the different steps of the treatment (cleaning, disinfection, lubrication) there are provided purging steps through compressed air, which are meant to empty the inside of the handpiece or to dry the outside of the handpiece and to prepare it for the subsequent step.

A first advantage of the present invention is linked to the rotation of the mechanical block for connecting said handpieces. The rotation of the block between two extreme positions allows a much improved usability by the human operator who has to insert the handpieces on the connectors. The fact that the block can take any angle between the two extreme positions facilitates the job.

A second advantage of the present invention is due to the fact that, thanks to the standardization of lubricating means spray can, many lubricating means on the market can be used for lubricating handpieces. This leads to two positive consequences. First, the specific lubricating means prescribed by the handpiece manufacturer can be used. Customary the handpiece manufacturer provides lubricant cans which contain a special formula for its handpieces; often the manufacturer binds the warranty to the use of the lubricant produced by the same company. Second, as CEFLA exports apparatuses all over the world, said spray cans can be found locally, without the need for CEFLA to ship a specific lubricant, shipping that undergoes the regulation concerning flammable products.

A third advantage, for the CDL embodiment only, is provided by the accessory for contrangles, which rotating the internal gear of the contrangle allows a much improved distribution of the cleaning and disinfecting means on the gears of the contrangle, improving the cleaning and the disinfection of contrangles.

Further advantages and properties of the present invention are disclosed in the following description, in which exemplary embodiments of the present invention are explained in detail based on the drawings:
- Figure 1: Axonometric view of a L/CDL device with closed front protection;
- Figure 2: Axonometric view of a L/CDL device with open front protection and handpiece in a first extreme position;
- Figure 3: Axonometric view of a L/CDL device with open front protection and handpiece in a second extreme position;
- Figure 4: Front view of a Midwest connector;
- Figure 5: Exemplary list of adapters for different connections;
- Figure 6: Axonometric view of a L/CDL device with open lid for the lubricating spray can;
- Figures 7A, 7B, 7C: Magnification of the portion housing the spray can in an axonometric view; transversal section of the housing portion of the spray can; axonometric, front and top view in assembled and exploded view of the detail of the coupling between spray can and connection;
- Figure 8: Axonometric vies of sliding shelf;
- Figures 9A, 9B, 9C: Lateral view, longitudinal section and exploded axonometric view of the accessory for connecting contrangles;
- Figure 10: Hydro-pneumatic diagram of the lubricating device L;
- Figure 11: Hydro-pneumatic diagram of the cleaning, disinfecting, lubricating device CDL.

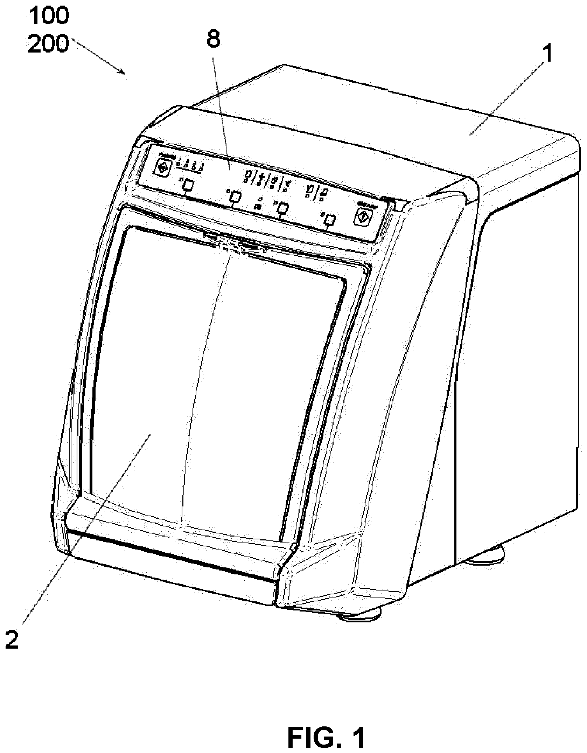

Figure 1 shows an L device 100/CDL device 200, comprising a body 1 and a front protection 2, here shown in its closed position. The devices L 100/CDL 200 further comprise an interface 8 for human operators, allowing to control said devices, and in the case of the CDL device 200, to choose the cycle to be performed (see below).

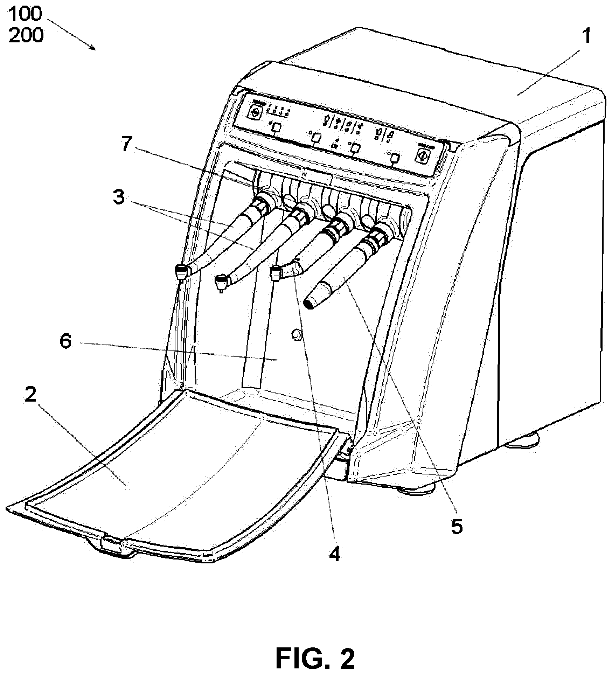

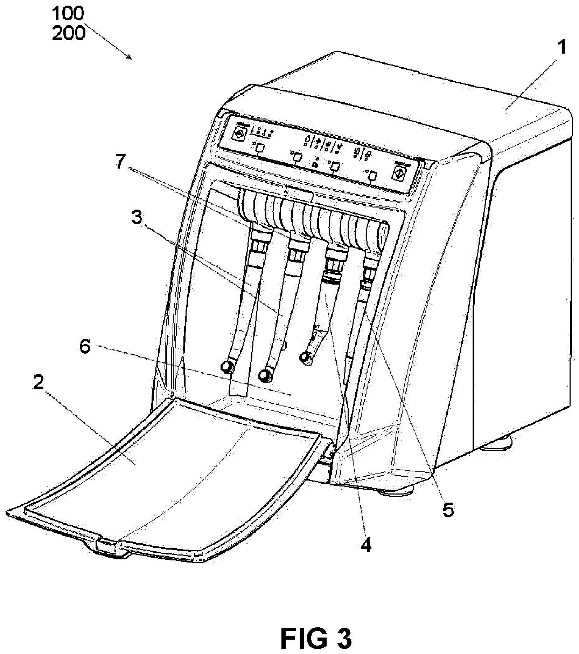

Figure 2 and Figure 3 show the device L100/CDL 200 with opened front protection. These Figures show that the opened front protection allows to reach a chamber 6 provided with a plurality of connectors 7, in particular four connectors 7, for connecting handpieces to be lubricated and possibly to be cleaned and disinfected. In the Figure, two turbines 3, one contrangle 4 and a dental scaler 5 for calculus removal are shown.

It is worth stating that the connectors 7 are all identical, and allow indifferently the connection with any kind of rotary or vibratory handpiece.

Said connectors 7 can be rotated upwards and downwards, around an axis which is parallel to ground, intended to facilitate human operators in inserting the handpieces 3, 4, 5 on said connectors 7. The comparison between Figures 2 and 3 shows the their two extreme positions:
- A first position for connecting handpieces, shown in Figure 2;
- A second operative position for lubricating and possibly cleaning and disinfecting handpieces, shown in Figure 3.

Of course, the cleaning, disinfecting and lubricating operation occur when the front protection is closed, as shown in Figure 1.

The angle between the first extreme position and the second extreme position of the connectors 7 for handpieces ranges 45-90°; in a preferred embodiment said angle is 52°. The allowed positions are the two said extreme positions and all the positions intermediate between those two extreme positions, in a continuous way.

Each connector 7 for handpieces 3, 4, 5 is arranged on a cylinder rotating around its longitudinal axis, parallel to ground. Said connectors rotate all at the same time.

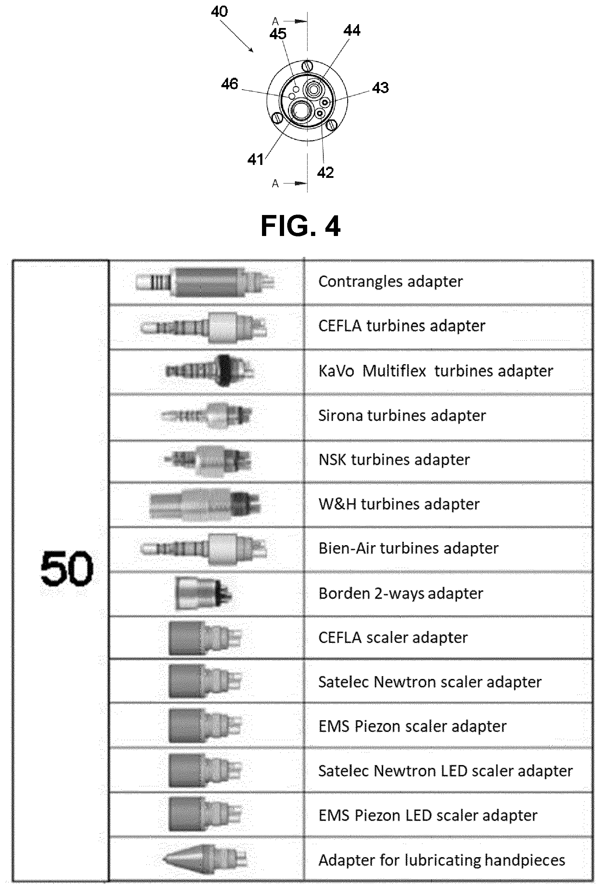

Figure 4 shows a front view of a screw Midwest connection 40. Said Midwest connections are well known in the art, and are a standard in dentistry (UNI EN 29168). They are provided with:
- A connector 41 for drainage;
- A connector 42 for cooling air (chip air);
- A connector 43 for cooling water;
- A connector 44 for motive air;
- A connector 45 for electrical contacts;
- A connector 45 for electrical contacts.

The Midwest connection is the main kind of screw connection used in dentistry. The dental handpieces 3, 4, 5 can be provided with different coupling connections wherein the channels for air, water and electricity are arranged in different ways; consequently, the devices L100/CDL 200 are provided with special adapters, which allow to lubricate and possibly clean and disinfect even handpieces provided with other kinds of connections.

Figure 5 shows an exemplary list of commercial adapters 50, to be interposed between the Midwest connectors 7 of the devices L 100/CDL 200 and the connectors of the single handpiece 3, 4, 5. Said commercial adapters are not part of the present invention and are not described further. The list is shown simply to recall that the connectors for dental handpieces are sundry. Often the handpiece manufacturer (e.g. KaVo, Sirona, W&H, NSK, Bien Air, Satelec, EMS, etc.) provides a proprietary connector which is provided on all the handpieces produced by said manufacturer. It is worth mentioning that all the vibratory handpieces need a special adapter 50 in order to be inserted on the Midwest connectors 7.

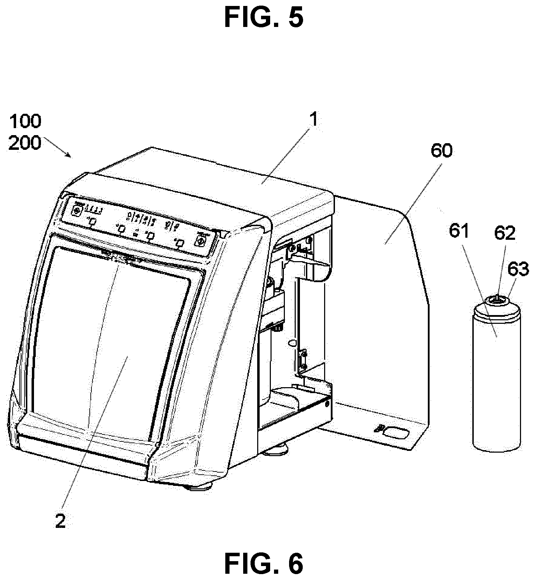

Figure 6 shows the devices L 100/CDL 200 with an opened lateral cover 60. The opening of said lateral cover 60 allows to reach a lateral chamber wherein both a lubricant spray can 61 is housed and, just in the CDL embodiment, also the reservoirs for the detergent and disinfectant means (see below) are housed.

As the Figure shows, the spray can 61 is provided with its customary tubular stem 62 for controlling the supply valve, but not with its customary nozzle (actuator) allowing to manually spray its content. Its nozzle (not shown) is removable without tools. One of the features of the present invention which improves its usability, is the possibility, as explained above, to use the devices 100, 200 for lubricating handpieces produced by any manufacturer. In this way the final user can use any lubricant spray can provided with a standard 1-inch collar 63.

Typically, spray cans are provided with said collar 63, which is the sealing connecting edge of the can lid to the supply valve of the can. Typically, those valves are normally closed, and can be only temporarily opened through pressure on the tubular stem 62, toward the inside of the spray can. When pressure on the tubular stem is released, the passage of the valve is automatically closed. Delving into details of the valve is not necessary, because this is not the object of the present invention. Nonetheless, the collar 63 is part of the structure of spray cans and of the combination with the features of the present invention.

The use of standard spray cans provided with a 1-inch collar 63 is possible thanks to a special clamp 70 provided for the spray can 61, and in particular for the collar 63, inside said the body 1 of devices 100, 200.

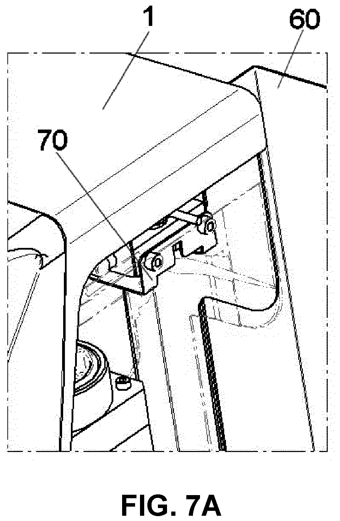

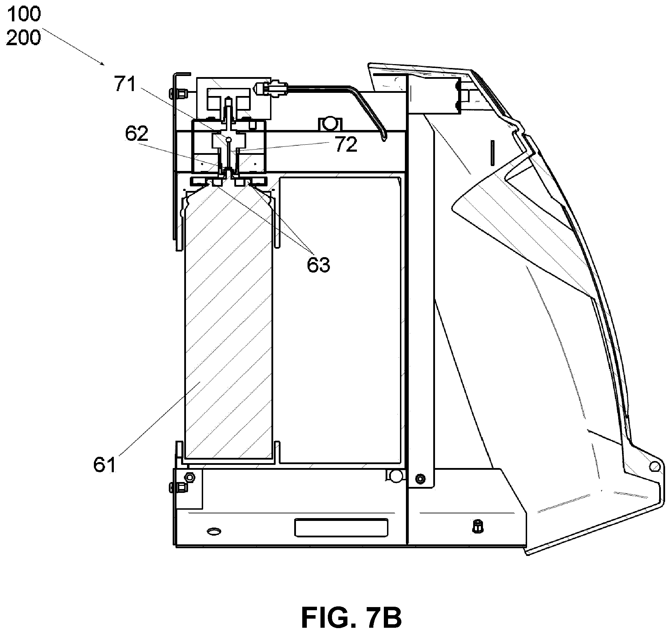

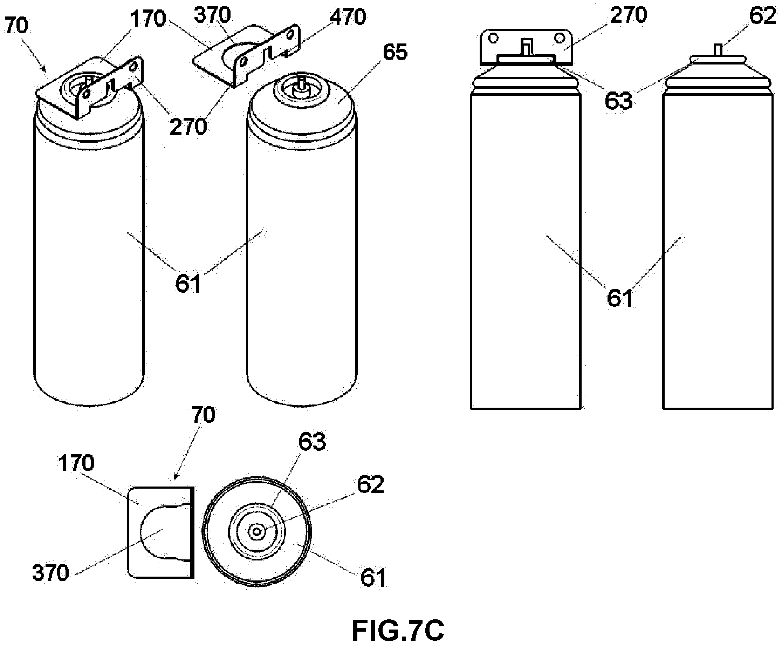

Figures 7 show the clamp 70 for a lubricant spray can 61 inside the body 1 of devices 100, 200. In particular, Figure 7A is a magnification of Figure 6 and shows an axonometric view of the body 1, while Figure 7B is a transversal section of the devices 100, 200. Figure 7C shows different axonometric, front and top views in assembled and exploded views of the coupling between the spray can 61 and its clamp 70, with the aim of clarifying the reciprocal positions and functioning.

Figure 7A shows the position of the clamp 70 housing the 1-inch collar 63 of the lubricating spray can 61. The connection is substantially allowed by an L-shaped clamp 70, provided with two branches 170, 270. Said branch 170 is oriented perpendicularly to the longitudinal axis of the spray can 61, while the branch 270 is oriented parallel to the longitudinal axis of the spray can. A slot 370, 470 (better visible in Figure 7C) is obtained in said clamp 70 and extends on both branches 170 and 270, without interruption between the two parts 370, 470 provided on one of the two branches 170, 270, respectively. The branch 270, parallel to the axis of the spray can, and in particular arranged e.g. in the vertical position in the shown embodiment, is fixed to the body 1 of the devices 100, 200. The portion 370 of the slot obtained in the branch 170 corresponds to the shape of the top head of the spray can 61, and in particular to the collar 63 and the stem 62, which shape is taken along the diametric section plan and parallel to the axis of the spray can 61. The portion 470 of the slot is connected without interruption with the portion 370 on the transversal branch 170 of said clamp 70, in particular parallel to the axis of the spray can, and is U-shaped, having a distance of the two U-shaped branches which is equal to the width of the portion 470 of the slot on the branch 270, and coinciding with the collar 63 of the spray can. The closed and arcuate portion of the U is closed substantially by a half-circle, corresponding to the radius of curvature of said collar 63 of the spray can. It is to be noted that obviously the dimensions and the radius of curvature are commensurate to the shape of the top head of the spray can, and in particular to the collar 63 and the stem 62, in the sense that they are slightly bigger, to the degree needed for the free insertion of the spray can 61 inside said L-shaped clamp 70 through said portions 370 and 470 of the slot. The portion 470 of the slot on the vertical branch 270 is meant to allow the introduction of the spray can 61 inside the portion 370 of the slot in the branch 170 of the clamp through a simple translation of the spray can in the direction perpendicular to its axis. The portion 370 of the slot, arranged in the horizontal branch 170 of the clamp 70, houses said collar 63 of the spray can 61, superimposed to a conical portion 65 of the spray can head, working as a top retention.

Said collar 63 is slightly larger than the lumen of the slot 70; gravity keeps the spray can 61 once the collar 63 is inserted inside said slot 70. When the collar 63 is placed, the stem 62 without its nozzle protrudes over said slot, contacting said actuator 62.

Over said stem 62 of the spray can 61 an actuator 72 is arranged (Figure 7B), which, pushed by compressed air coming from an electro valve controlled by an electronic board pushes said stem 63, causing the outlet of the lubricating means from said spray can 61; said lubricant is then channelled inside its tubing through a hole 71.

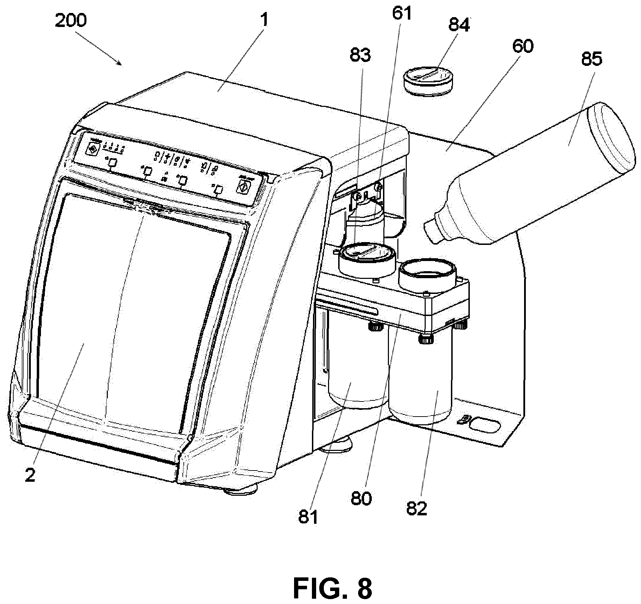

Figure 8 shows the device CDL 200 provided with a sliding shelf 80 in its extracted position. Said sliding shelf 80 is provided with two slots, which hold two reservoirs 81, 82 provided for a cleaning and a disinfecting means, respectively. Each reservoir 81, 82 is provided with a pressure lid 83, 84. The content of said reservoirs 81, 82 can be directly refilled using a container 85 inside which said products are sold.

The shelf 80 slides and is extracted from the device CDL 200 in order to refill the products when said device 200 is not in use. The sliding shelf 80 slides laterally with respect to said spray can 61, visible in the Figure.

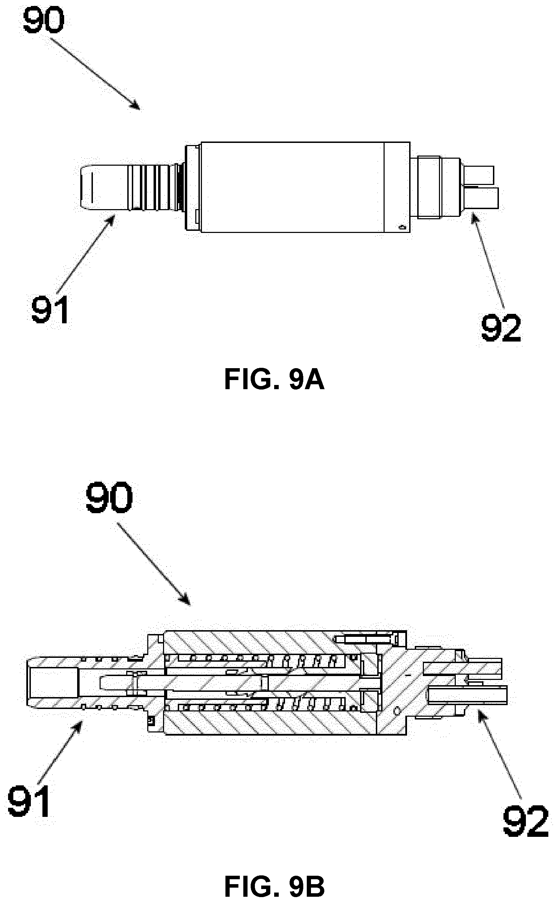

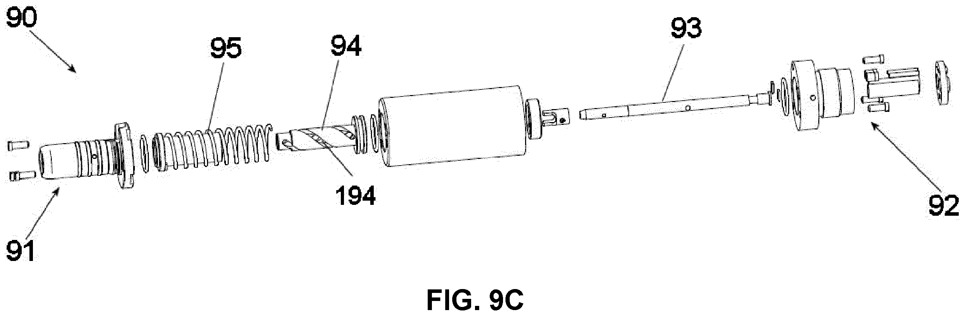

Figures 9 show a special adapter 90 for contrangles 4, conceptually analogous to the adapters 50 shown in Figure 5. In particular, Figure 9A shows said adapter 90 in a side view, Figure 9B shows the adapter 90 in a longitudinal section cut according to plane AA shown in Figure 4, while Figure 9C shows said adapter in an axonometric exploded view.

Said adapter 90 is provided with a cylindrical shape; at its first end there is provided a connection 91 for a contrangle, while at its second end there is provided a Midwest connection, allowing the connection of the adapter 90 on any of the connectors 7 for the handpieces. Figure 9C shows the internal components of said adapter, comprising a shaft 93, a portion 94 mobile in the axial direction, and a spring 95.

In the CDL embodiment 200 only, the inside of handpieces 3, 4, 5, first undergoes a cleaning and a disinfection, and subsequently a lubrication (for rotary handpieces only); in the internal channels of the handpieces first a detergent is circulated, withdrawn from its reservoir 81, and secondly a disinfectant withdrawn from its reservoir 82.

In the case of vibratory handpieces, which are not lubricated, when the control for supplying lubricant is actuated, which reaches all connectors 7 for handpieces, the lubricant is drained inside the chamber 6, thanks to the presence of a hole in the adapter 50 allowing the connection between the vibratory handpiece and said connector 7.

In order to ensure that all the lumens of the channels of said contrangle 4 are wetted by the detergent and the disinfectant, the internal gear of said contrangle must be rotated. Said function is performed by the adapter 90.

The detergent or the disinfectant withdrawn by their respective reservoirs 81, 82 is forced inside said adapter 90 with compressed air; in particular, the detergent/disinfectant mixed with compressed air enters from the connector 42 of the Midwest connector, passing through the cavity formed by the mantel wall of the shaft 93 and the body of the adapter 90. The pressurized air pushes forward the mobile portion 94, which turns the shaft 93 thanks to a constraint due to a (not shown) radial pin fixed to the shaft 93, which engages in a helical groove 194 of the mobile portion 94. The mobile portion 94 is dragged forward, guided along an axial rectilinear path toward the connector 91 for the contrangle, for a stroke of about 15 mm. The axial movement of the mobile portion 94 determines the rotation of said shaft 93 of about one turn, thanks to said helical groove 194 and to the radial pin of the shaft 93 engaged in said helical groove 194. Once the supply of compressed air is stopped, the spring 95 determines a backward movement of about one turn of the shaft 93 in the opposed direction. This double rotation determines a better distribution of the detergent/disinfectant on the contrangle gears.

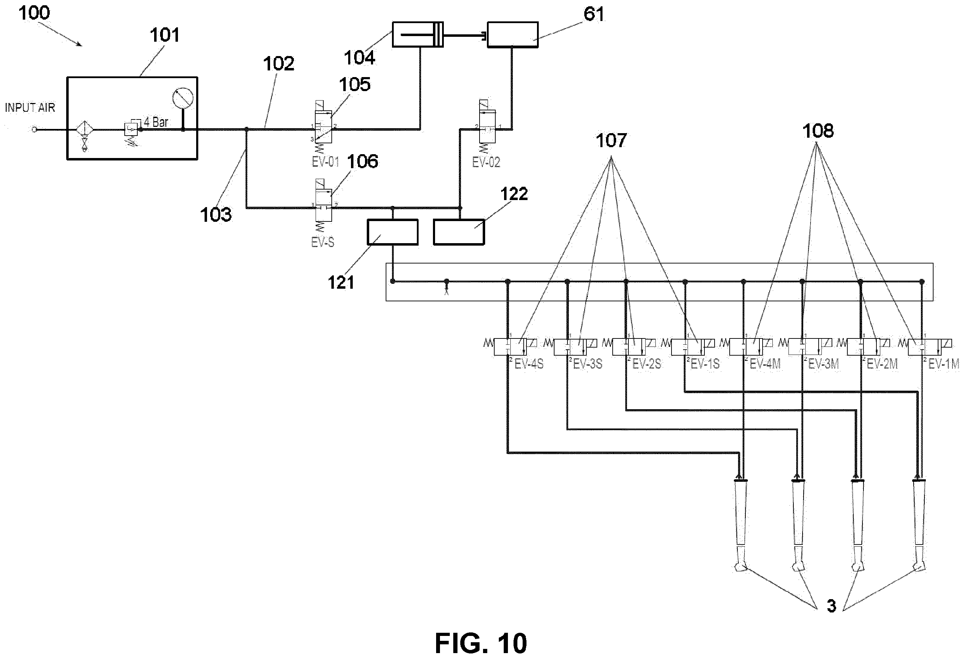

Figure 10 shows a hydro-pneumatic diagram of the device L 100. The device L 100 must be connected to an external compressed air source (INPUT AIR) which works at 4-6 bar; said compressed air is stabilized at a pressure of 4 bar through a pressure regulator 101, which also works a filter for compressed air. Downstream said pressure regulator, compressed air is subdivided into two branches 102, 103, the first branch 103 of which brings compressed air directly to handpieces for purging, while the second branch 102 passes through a cylinder 104 actuating the actuator 72 and the lubricant spray can 61. The circuit moreover comprises an oil sensor 121 and a pressure sensor 122.

A first group of electro valves 107, one for each handpiece, controls air for spray channels, while a second group of electro valves 108, one for each handpiece, controls the main air line for handpieces.

Suitably opening and closing said electro valves 105, 106, 107, 108, lubricant can be supplied to handpieces 3 inserted on connectors 7, which are lubricated one at a time, in series. For the sake of simplicity, the Figure shows four turbines 3, but it is understood that there can be provided any kind of rotary handpiece. The lubricant passes through rotary handpieces 3, leaving from the head of the handpiece and draining into said chamber 6.

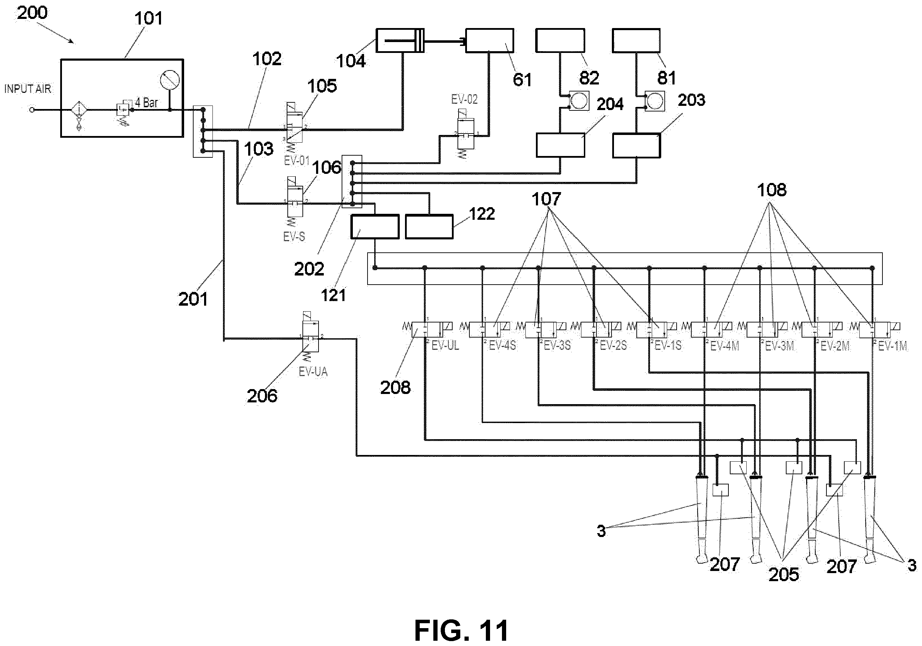

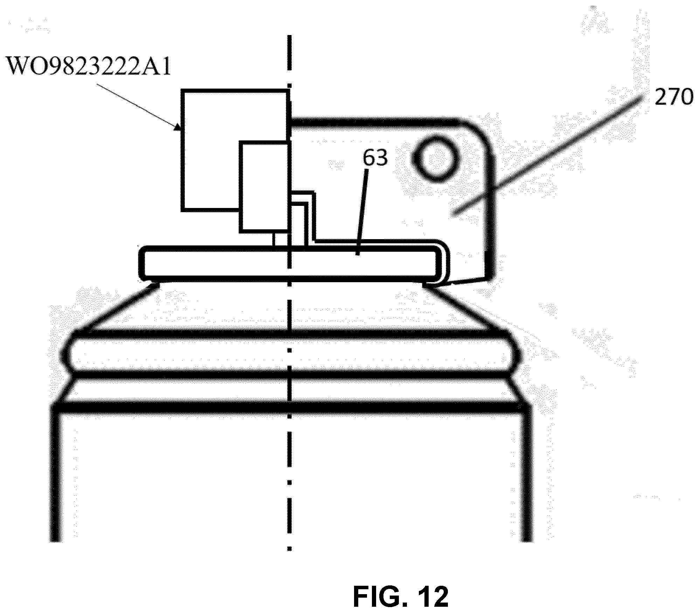

Figure 11 shows a hydro-pneumatic diagram of the device CDL 200; for the sake of simplicity, in this Figure, too, four turbines 3 are shown, it being understood that there can be provided any kind of rotary or vibratory handpiece. Concerning lubrication, the CDL device 200 works in the same above-described way as L device 100.

Concerning the supply of detergent and disinfectant inside handpieces, it works in a way similar to that of the lubricant, even if in this case the supply is due to a pair of pumps 203, 204. In this case, too, detergent and disinfectant are supplied in series, one at a time for each handpiece, in a mutually exclusive way passing through a distributor 202. Before supplying the detergent/disinfectant to each handpiece 3, the detergent/disinfectant is supplied through three nozzles 205 arranged inside the chamber 6 at the same time as compressed air supplied by two nozzles 207, which are arranged in said chamber 6, too. Said compressed air is meant to create a whirling motion of the disinfectant, which in this way is distributed on the external surface of said handpieces 3. An electro valve 206 is intended for regulating the afflux of air to said nozzles 207. An electro valve 208 is meant to regulate the afflux of air to said nozzles 205, 207.

Said distributor 202 mixes the compressed air coming from the outside alternatively with the lubricant coming from the spray can 61, with the detergent coming from the reservoir 81, or with the disinfectant coming from reservoir 82.

As said, in the case of the vibratory handpieces, the lubricant is drained through a special (not shown) hole of the adapter 50 and drained in the chamber 6.

A typical cycle of the device L 100 comprises the following steps:
a) Insertion of the handpieces 3, 4 on the connector 7 by a human operator, after they have been manually cleaned on their outside; the human operator interposing an adapter 50 according to need;
b) Closing of the front protection 2 by the human operator;
c) Purging of the internal channels of the handpieces through compressed air;
d) Lubrication of the air channel which brings air to the rotary parts of handpieces;
e) Purging of all the internal channels of the handpieces to remove the excess of lubricant;
f) Opening of the front protection 2 and extraction of the lubricated handpieces 3, 4 by the human operator.

Typically the CDL devices 200 are pre-programmed, and allow to perform different cycles of lubrication, cleaning and disinfection, as desired. In particular, the CDL device 200 allows to choose among four different pre-programmed cycles:
1. Purging - cleaning - purging - disinfection - purging - lubrication - purging
2. Purging - cleaning - purging
3. Purging - lubrication - purging
4. Purging

The cycle 1, the most complex provided on the device CDL 200, comprises the following steps:
a) Insertion of the handpieces 3, 4, 5 on the connectors 7 by a human operator, after they have been manually cleaned on their outside; the human operator interposing an adapter 50 or 90 according to need;
a) Closing of the front protection 2 by the human operator;
b) Selection of the desired pre-programmed cycle by the human operator through the interface 8;
c) Purging of the internal channels of the handpieces through compressed air;
d) Cleaning of the external surface of the handpieces 3, 4, 5 through supply of the detergent;
e) Cleaning of the internal channels of the handpieces 3, 4, 5 through supply of the detergent;
f) The detergent is allowed to act for the time indicated by its manufacturer;
g) Purging of the internal channels of the handpieces through compressed air;
h) Disinfection of the external surface of the handpiece by spraying the disinfectant on the surface of the handpieces inside said chamber 6;
i) Disinfection of the internal channels of the handpieces 3, 4, 5 through supply of the disinfectant;
j) The disinfectant is allowed to act for the time indicated by its manufacturer;
k) Purging of the internal channels of the handpieces through compressed air;
l) Supply of compressed air on the outside of the handpieces 3, 4, 5 in order to remove said disinfectant;
m) Lubrication of the air channel that brings air to the rotary parts of the handpieces 3, 4;
n) Purging of all internal channels in order to remove the excess lubricant;
o) Opening of the front protection 2 and extraction of the lubricated handpieces 3, 4, 5 by the human operator.

In order to safeguard patients' health, dental practices are provided with a quality system which provides a record of the treatments performed on the re-usable devices, like dental handpieces. Therefore, it is known that treatment devices are provided with an internal memory allowing to record the performed cycles. The information concerning said cycles is downloadable on a USB wand, which can be connected to the device L 100/ CDL 200.

| | | | |
|---|---|---|---|
| 1 | device body | 90 | contrangle adapter |
| 2 | front protection | 91 | contrangle connector |
| 3 | turbine | 92 | Midwest connector |
| 4 | contrangle | 93 | shaft |
| 5 | dental scaler | 94 | axial mobile portion |
| 6 | chamber | 95 | spring |
| 7 | connectors | 100 | L device |
| 8 | human operator interface | 101 | pressure regulator |
| 40 | Midwest connector | 102 | lubricant branch |
| 41 | drainage connector | 103 | compressed air branch |
| 42 | air spray connector | 104 | cylinder |
| 43 | cooling water connector | 105 | electro valve |
| 44 | motive air connector | 106 | electro valve |
| 45 | electrical connector | 121 | oil sensor |
| 46 | electrical connector | 122 | pressure sensor |
| 50 | adapter | 170 | horizontal branch |
| 60 | lateral cover | 194 | helical groove |
| 61 | lubricant spray can | 200 | CDL device |
| 62 | stem | 201 | third branch |
| 63 | collar | 202 | distributor |
| 65 | conical portion of spray can | 203 | pump |
| 70 | slot for the collar | 204 | pump |
| 71 | lubricant hole | 205 | nozzle |
| 72 | actuator | 206 | electro valve |
| 80 | sliding shelf | 207 | nozzle |
| 81 | detergent reservoir | 208 | electro valve |
| 82 | disinfectant reservoir | 270 | vertical branch |
| 83 | detergent lid | 370 | horizontal slot |
| 84 | disinfectant lid | 470 | vertical slot |
| 85 | original container | | |

## Claims

1. Desktop device (200) for cleaning, disinfecting and lubricating dental handpieces (3, 4, 5) comprising connectors (7) for the insertion of said handpieces placed inside an openable chamber (6), wherein said connectors are provided with a Midwest connection; said device comprising an adapter (90) for contrangles to be interposed between said contrangles (4) and said connectors (7); said adapter (90) being provided with a cylindrical shape; at a first end of said adapter there being provided a connector (91) for a contrangle, while at a second end of said adaptor there being provided a Midwest connector (92);
**characterized in that**
said adapter (90) comprises the following components
- a shaft (93) coaxially inserted in
- an axially mobile cylindrical portion (94) in its turn surrounded by
- a spring (95),
said components (93, 94, 95) being surrounded by the outside housing of said adapter (90) and wherein
- said shaft (93) rotates with respect to the cylindrical portion (94);
- said cylindrical portion (94) and said shaft (93) are engaged through a threaded rod-lead screw connection;
- said shaft (93) can be rotatably coupled with gears of a contrangle (4) when said adapter (90) is coupled to said contrangle (4).

2. Desktop device (200) for cleaning, disinfecting and lubricating dental handpieces (3, 4, 5) according to claim 1, wherein when compressed air passes through said adapter (90), said shaft (93) rotates, allowing a better distribution of a detergent/disinfectant on internal gears of said contrangle (4).

3. Desktop device (200) for cleaning, disinfecting and lubricating dental handpieces (3, 4, 5) according to claim 1 or 2, comprising an adapter, wherein, when said handpieces (3, 4, 5) are not provided with a Midwest connector, between said handpiece (3, 4, 5) and the connector (7) said adapter is interposed (50).

4. Desktop device (200) for cleaning, disinfecting and lubricating dental handpieces (3, 4, 5) according to one or more of the preceding claims, wherein the rotary handpieces are chosen from the group consisting of turbines (3) and contrangles (4), while vibratory handpieces are dental calculus scalers (5).

5. Desktop device (200) for cleaning, disinfecting and lubricating dental handpieces (3, 4, 5) according to one or more of the preceding claims comprising a circuit, wherein the circuit comprises
- a compressed air inlet;
- a pressure stabilizer (101);
- a first branch (103), provided with an electro valve (106) directly connected to the connectors (7) for dental handpieces;
- a second branch (102), provided with an electro valve (105) connected to the connectors (7) for dental handpieces through a hole (71) supplying a lubricating means, contained inside a spray can (61);
wherein the opening/closing of said electro valves (103, 106) supplies to said handpieces (3, 4) alternatively compressed air only or compressed air mixed with lubricant means; said lubricant passing through said handpieces (3, 4) to be eventually drained into said chamber (6).

6. Desktop device (200) for cleaning, disinfecting and lubricating dental handpieces (3, 4, 5) according to claim 5, wherein a hole provided in said adapter (50) drains said lubricant directly in said chamber (6), without passing through vibratory handpieces (5).

7. Desktop device (200) for cleaning, disinfecting and lubricating dental handpieces (3, 4, 5) according to claim 5 or 6, wherein the circuit, in addition to the components according to claim 5, comprises:
- a further branch (201) provided with an electro valve (206), connected to special nozzles (205);
- a detergent reservoir (82) actuated by a pump (204);
- a disinfectant reservoir (81) actuated by a pump (203);
wherein the opening/closing of said electro valves (105, 106, 206) supplies said handpieces alternatively with lubricant, detergent, disinfectant withdrawn from their respective reservoirs (61, 82, 81).

8. Desktop device (200) for cleaning, disinfecting and lubricating dental handpieces (3, 4, 5) according to claim 7, wherein the circuit, in addition to the components according to claim 8, further comprises nozzles (205) arranged in said chamber (6) supplying detergent/disinfectant to the outside of said handpieces (3, 4, 5) and nozzles (207), also arranged in said chamber (6), supplying compressed air inside said chamber (6), in order to generate a whirling motion of said detergent/disinfectant.

9. Desktop device (100) for lubricating or desktop device (200) for cleaning, disinfecting and lubricating dental handpieces (3, 4, 5) according to one or more of the preceding claims wherein said devices comprise an USB slot for connection to an USB wand provided for downloading the data concerning lubricating cycles or cleaning, disinfection and lubricating cycles performed on said handpieces (3, 4, 5).

## Patentansprüche

1. Tischvorrichtung (200) zum Reinigen, Desinfizieren und Schmieren von zahnärztlichen Handstücken (3, 4, 5), umfassend Anschlüsse (7) im Inneren einer öffenbaren Kammer (6) zum Einsetzen der Handstücke, wobei die Anschlüsse mit einem Midwest-Anschluss versehen sind; wobei die Vorrichtung einen Adapter (90) für Winkelstücke umfasst, der zwischen die Winkelstücke (4) und die Anschlüsse (7) zwischengeschaltet ist; wobei der Adapter (90) mit einer zylindrischen Form versehen ist; wobei an einem ersten Ende des Adapters ein Anschluss (91) für ein Winkelstück vorgesehen ist, während an einem zweiten Ende des Adapters ein Midwest-Anschluss (92) vorgesehen ist;
**dadurch gekennzeichnet, dass**
der Adapter (90) die folgenden Komponenten umfasst
- eine Welle (93), die koaxial eingesetzt ist in
- einen axial beweglichen zylindrischen Abschnitt (94), der seinerseits von
- einer Feder (95) umgeben ist,
wobei die Komponenten (93, 94, 95) von dem Außengehäuse des Adapters (90) umgeben sind und wobei
- die Welle (93) sich in Bezug auf den zylindrischen Abschnitt (94) dreht;
- der zylindrische Abschnitt (94) und die Welle (93) durch eine Gewindestange-Spindelmutterverbindung miteinander verbunden sind;
- die Welle (93) drehbar mit Getrieben eines Winkelstücks (4) gekoppelt werden kann, wenn der Adapter (90) mit dem Winkelstück (4) gekoppelt ist.

2. Tischvorrichtung (200) zum Reinigen, Desinfizieren und Schmieren von zahnärztlichen Handstücken (3, 4, 5) nach Anspruch 1, wobei, wenn Druckluft durch den Adapter (90) strömt, die Welle (93) rotiert, wodurch eine bessere Verteilung eines Reinigungs-/Desinfektionsmittels auf die inneren Getriebe des Winkelstücks (4) ermöglicht wird.

3. Tischgerät (200) zum Reinigen, Desinfizieren und Schmieren von zahnärztlichen Handstücken (3, 4, 5) nach Anspruch 1 oder 2, umfassend einen Adapter, wobei, falls die Handstücke (3, 4, 5) nicht mit einem Midwest-Anschluss versehen sind, der Adapter zwischen dem Handstück (3, 4, 5) und dem Anschluss (7) zwischengeschaltet ist (50).

4. Tischvorrichtung (200) zum Reinigen, Desinfizieren und Schmieren von zahnärztlichen Handstücken (3, 4, 5) nach einem oder mehreren der vorhergehenden Ansprüche, wobei die rotierenden Handstücke aus der Gruppe bestehend aus Turbinen (3) und Winkelstücken (4) ausgewählt sind, während die vibrierenden Handstücke Zahnsteinentferner (5) sind.

5. Tischvorrichtung (200) zum Reinigen, Desinfizieren und Schmieren von zahnärztlichen Handstücken (3, 4, 5) nach einem oder mehreren der vorhergehenden Ansprüche, umfassend einen Kreislauf, wobei der Kreislauf umfasst
- einen Drucklufteinlass;
- einen Druckstabilisator (10 1);
- einen ersten Zweig (103), der mit einem unmittelbar mit den Anschlüssen (7) für zahnärztliche Handstücke verbundenen Elektroventil (106) versehen ist;
- einen zweiten Zweig (102), der mit einem Elektroventil (105) versehen ist, das mit den Anschlüssen (7) für zahnärztliche Handstücke durch eine Öffnung (71) verbunden ist, die ein in einer Sprühdose (61) enthaltenes Schmiermittel zuführt,
wobei das Öffnen/Schließen der Elektroventile (103, 106) den Handstücken (3, 4) wahlweise nur Druckluft oder mit Schmiermittel gemischte Druckluft zuführt; wobei das Schmiermittel durch die Handstücke (3, 4) hindurchgeht, um schließlich in die Kammer (6) abgelassen zu werden.

6. Tischvorrichtung (200) zum Reinigen, Desinfizieren und Schmieren von zahnärztlichen Handstücken (3, 4, 5) nach Anspruch 5, wobei ein in dem Adapter (50) vorgesehenes Loch das Schmiermittel unmittelbar in die Kammer (6) ablässt, ohne Durchgang durch vibrierende Handstücke (5).

7. Tischvorrichtung (200) zum Reinigen, Desinfizieren und Schmieren von zahnärztlichen Handstücken (3, 4, 5) nach Anspruch 5 oder 6, wobei der Kreislauf zusätzlich zu den Komponenten nach Anspruch 5 umfasst:
- einen weiteren Zweig (201) mit einem Elektroventil (206), das mit speziellen Düsen (205) verbunden ist;
- einen Reinigungsmittelbehälter (82), der durch eine Pumpe (204) betätigt wird;
- einen Desinfektionsmittelbehälter (81), der durch eine Pumpe (203) betätigt wird;
wobei das Öffnen/Schließen der Elektroventile (105, 106, 206) die Handstücke wahlweise mit Schmiermittel, Reinigungsmittel und Desinfektionsmittel versorgt, die aus ihren jeweiligen Behältern (61,82,81) entnommen werden.

8. Tischvorrichtung (200) zum Reinigen, Desinfizieren und Schmieren von zahnärztlichen Handstücken (3, 4, 5) nach Anspruch 7, wobei der Kreislauf zusätzlich zu den Komponenten nach Anspruch 8 weiterhin in der Kammer (6) angeordnete Düsen (205) umfasst, die Reinigungs-/Desinfektionsmittel an die Außenseite der Handstücke (3, 4, 5) zuführen, und Düsen (207), die ebenfalls in der Kammer (6) angeordnet sind und Druckluft ins Innere der Kammer (6) liefern, um eine Wirbelbewegung des Reinigungs-/Desinfektionsmittels zu erzeugen.

9. Tischvorrichtung (100) zum Schmieren oder Tischvorrichtung (200) zum Reinigen, Desinfizieren und Schmieren von zahnärztlichen Handstücken (3, 4, 5) nach einem oder mehreren der vorhergehenden Ansprüche, wobei die Vorrichtungen einen USB-Steckplatz zum Anschluss eines USB-Sticks umfassen, der zum Herunterladen der die an den Handstücken (3, 4, 5) durchgeführten Schmierzyklen oder Reinigungs-, Desinfektions- und Schmierzyklen betreffenden Daten vorgesehen ist.

## Revendications

1. Dispositif d'établi (200) pour nettoyer, désinfecter et lubrifier des pièces à main dentaires (3, 4, 5) comprenant des connecteurs (7) pour l'insertion desdites pièces à main placés à l'intérieur d'une chambre ouvrable (6), dans lequel lesdits connecteurs sont fournis avec un connecteur Midwest; ledit dispositif comprenant un adaptateur (90) pour des contre-angles à interposer entre lesdits contre-angles (4) et lesdits connecteurs (7); ledit adaptateur (90) étant fourni de forme cylindrique; un connecteur (91) pour un contre-angle étant fourni en correspondance d'une première extrémité dudit adaptateur, tandis qu'un connecteur Midwest (92) est fourni en correspondance d'une seconde extrémité dudit adaptateur;
**caractérisé en ce que**
ledit adaptateur (90) comprend les composants suivants:
- un arbre (93) inséré de manière coaxiale dans
- une portion cylindrique (94) mobile de manière coaxiale, à son tour entourée d'un
- ressort (95),
lesdits composants (93, 94, 95) étant entourés par le logement externe dudit adaptateur (90) et dans lequel
- ledit arbre (93) tourne par rapport à la portion cylindrique (94);
- ladite portion cylindrique (94) et ledit arbre (93) sont engagées au moyen d'une connexion écrou - tige filetée;
- ledit arbre (93) peut être couplé en rotation avec des engrenages d'un contre-angle (4) lorsque ledit adaptateur (90) est couplé audit contre-angle (4).

2. Dispositif d'établi (200) pour nettoyer, désinfecter et lubrifier des pièces à main dentaires (3, 4, 5) selon la revendication 1, dans lequel ledit arbre (93) tourne, permettant une meilleure distribution d'un détergent/désinfectant sur des engrenages internes dudit contre-angle (4), lorsque l'air comprimé passe à travers ledit adaptateur (90).

3. Dispositif d'établi (200) pour nettoyer, désinfecter et lubrifier des pièces à main dentaires (3, 4, 5) selon la revendication 1 ou 2, comprenant un adaptateur,
dans lequel ledit adaptateur est interposé (50) entre ladite pièce à main (3, 4, 5) et ledit connecteur (7) lorsque lesdites pièces à main (3, 4, 5) ne sont pas fournies avec un connecteur Midwest.

4. Dispositif d'établi (200) pour nettoyer, désinfecter et lubrifier des pièces à main dentaires (3, 4, 5) selon l'une ou plusieurs des revendications précédentes, dans lequel les pièces à main rotatives sont sélectionnées parmi le groupe consistant des turbines (3) et des contre-angles (4), tandis que les pièces à main vibrantes sont des détartreurs de tartre dentaire (5).

5. Dispositif d'établi (200) pour nettoyer, désinfecter et lubrifier des pièces à main dentaires (3, 4, 5) selon l'une ou plusieurs des revendications précédentes, comprenant un circuit, dans lequel le circuit comprend:
- une entrée d'air comprimé;
- un stabilisateur de pression (101);
- une première branche (103) fournie avec une électrovanne (106) directement connectée aux connecteurs (7) pour pièces à main dentaires;
- une second branche (102) fournie avec une électrovanne (105) connectée aux connecteurs (7) pour pièces à main dentaires par un trou (71) fournissant un moyen de lubrification contenu à l'intérieur d'une bombe aérosol (61);
dans lequel l'ouverture/la fermeture desdites électrovannes (103, 106) fournit auxdites pièces à main (3, 4) alternativement de l'air comprimé uniquement ou de l'air comprimé mélangé à des moyens de lubrifications; ledit lubrifiant passant à travers lesdites pièces à main (3, 4) pour être ensuite drainé dans ladite chambre (6).

6. Dispositif d'établi (200) pour nettoyer, désinfecter et lubrifier des pièces à main dentaires (3, 4, 5) selon la revendication 5, dans lequel un trou fourni dans ledit adaptateur (50) draine ledit lubrifiant directement dans ladite chambre (6), sans passer à travers les pièces à main vibrantes (5).

7. Dispositif d'établi (200) pour nettoyer, désinfecter et lubrifier des pièces à main dentaires (3, 4, 5) selon la revendication 5 ou 6, dans lequel le circuit, en plus des composants selon la revendication 5, comprend:
- une autre branche (201) fournie avec une électrovanne (206) connectée à des buses spéciales (205);
- un réservoir de détergent (82) actionné par une pompe (204);
- un réservoir de désinfectant (81) actionné par une pompe (203);
dans lequel l'ouverture/la fermeture desdites électrovannes (105, 106, 206) fournit auxdites pièces à main alternativement du lubrifiant, du détergent, du désinfectant prélevés dans leurs réservoirs respectifs (61, 82, 81).

8. Dispositif d'établi (200) pour nettoyer, désinfecter et lubrifier des pièces à main dentaires (3, 4, 5) selon la revendication 7, dans lequel le circuit, en plus des composants selon la revendication 8, comprend en outre des buses (205) disposées dans ladite chambre (6) fournissant le détergent/le désinfectant à l'extérieur desdites pièces à main (3, 4, 5) et des buses (207), également disposées dans ladite chambre (6), fournissant de l'air comprimé à l'intérieur de ladite chambre (6), de sorte à générer un mouvement tourbillonnant dudit détergent/ désinfectant.

9. Dispositif d'établi (100) pour lubrifier ou un dispositif d'établi (200) pour nettoyer, désinfecter et lubrifier des pièces à main dentaires (3, 4, 5) selon l'une ou plusieurs des revendications précédentes, dans lequel lesdits dispositifs comprennent une connexion USB pour la connexion à une clé USB fournie pour télécharger les données concernant les cycles de lubrification ou de nettoyage, de désinfection et les cycles de lubrification effectués sur lesdites pièces à main (3, 4, 5).
